**Europäisches Patentamt**

**European Patent Office**

**Office européen des brevets**

(11) Veröffentlichungsnummer: **0 100 074**
**A1**

# EUROPÄISCHE PATENTANMELDUNG

(21) Anmeldenummer: **83107213.7**

(22) Anmeldetag: **22.07.83**

(51) Int. Cl.³: **C 07 C 9/04,** C 07 C 7/20
// B01J19/14, C10L3/00

(30) Priorität: **28.07.82 DE 3228092**

(43) Veröffentlichungstag der Anmeldung: **08.02.84**
**Patentblatt 84/6**

(84) Benannte Vertragsstaaten: **BE DE FR LU NL**

(71) Anmelder: **Ruhrgas Aktiengesellschaft,**
**Huttropstrasse 60 Postfach 10 32 52,**
**D-4300 Essen 1 (DE)**

(72) Erfinder: **Vogel, Gerhard, Dr.-Ing., Auf dem Holleter 33,**
**D-4300 Essen 1 (DE)**

(54) **Einrichtung zum Inertisieren von Erdgas.**

(57) Offenbart wird eine Einrichtung zum Inertisieren von Erdgas unter Verwendung von Rauchgas, mit einer Verbrennungsvorrichtung (1) zum Erzeugen des Rauchgases, mit einer Kühlvorrichtung (3) zu dessen Kühlung und mit einem Verdichter (6) zum Verdichten des Rauchgases auf den für das Zumischen zum Erdgas erforderlichen Druck, wobei diese Einrichtung dadurch gekennzeichnet ist, daß die Kühlvorrichtung (3) als Austreiber einer Absorptionskältevorrichtung (4) ausgebildet ist, deren Verdampfer (7) dem Verdichter (6) nachgeschaltet ist.

Ruhrgas AG Essen

Essen, 06.07.1982
tatp hs-di 447

Einrichtung zum Inertisieren von Erdgas

Die Erfindung betrifft eine Einrichtung zum Inertisieren von Erdgas unter Verwendung von Rauchgas, mit einer Verbrennungseinrichtung zum Erzeugen des Rauchgases, mit einer Kühlvorrichtung zu dessen Kühlung und mit einem Verdichter zum Verdichten des Rauchgases auf den für das Zumischen zum Erdgas erforderlichen Druck.

Bei einer bekannten Einrichtung dieser Art enthält das aus dem Verdichter kommende Rauchgas bei seiner Zumischung zum Erdgas noch ungenutzte Restwärme. Auch muß dieses Rauchgas, obwohl es vor dem Verdichter bereits getrocknet wurde, unter Umständen hinter dem Verdichter einer weiteren Trocknung unterworfen werden. Es treten also Verluste auf, die die Wirtschaftlichkeit der Einrichtung beeinträchtigen.

Dementsprechend liegt der Erfindung die Aufgabe zugrunde, die eingangs genannte Einrichtung in ihrer Wirtschaftlichkeit zu verbessern.

Zur Lösung dieser Aufgabe ist die Einrichtung nach der Erfindung dadurch gekennzeichnet, daß die Kühlvorrichtung als Austreiber einer Absorptionskältevorrichtung ausgebildet ist, deren Verdampfer dem Verdichter nachgeschaltet ist.

Auf diese Weise läßt sich die Restwärme des Rauchgases hinter dem Verdichter entnehmen, und zwar in Koppelung mit der dem Verdichter vorgeschalteten Kühlung. Die dem Verdichter nachgeschaltete Abkühlung kann bis unter den Taupunkt des Rauchgases gehen. Dadurch
erfolgt eine gleichzeitige Trocknung des Rauchgases.

Die Absorptionskältevorrichtung arbeitet mit hohem Wirkungsgrad, so
daß neben der Optimierung des Prozesses eine maxixmale Menge an
Nutzwärme abgeführt werden kann.

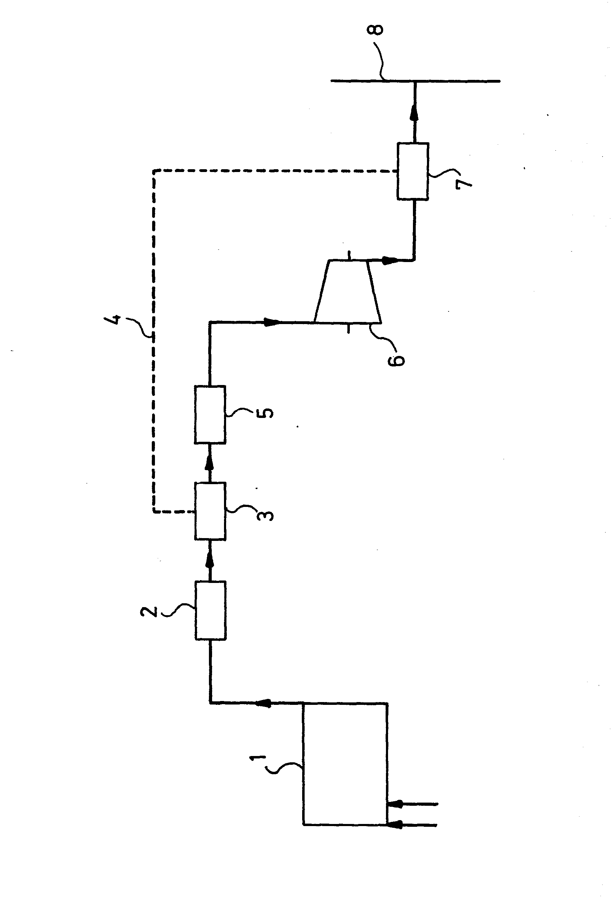

Die Erfindung wird im folgenden anhand eines bevorzugten Ausführungsbeispiels im Zusammenhang mit der beiliegenden Zeichnung näher
erläutert. Die Figur zeigt ein Blockschaltbild des Ausführungsbeispiels.

Demnach ist eine Verbrennungsvorrichtung 1 vorgesehen, bei der es
sich um eine Dampfkesselanlage oder um eine mit geschlossenem
Kreislauf arbeitende Gasturbine handeln kann. Die Verbrennungsvorrichtung wird auf etwa stöchiometrische oder etwas unterstöchiometrische Verbrennung eingestellt und erzeugt ein im wesentlichen
luft- bzw. sauerstofffreies Rauchgas, welches sich als Inertgas für
die Zumischung zum Erdgas eignet. Das Rauchgas gelangt aus der
Verbrennungsvorrichtung 1 in eine Reinigungsvorrichtung 2 und
sodann in eine Kühlvorrichtung 3, die als Austreiber einer Absorptionskältevorrichtung, angedeutet durch die gestrichelte Linie 4,
ausgebildet ist. An den Austreiber schließt sich eine Trocknungsvorrichtung 5 an, in der gegenbenenfalls auch noch eine weitere
Kühlung stattfinden kann. Sodann gelangt das Rauchgas zu einem
Verdichter 6, der es durch einen Verdampfer 7 der Absoprtionskältevorrichtung 4 in eine Erdgasleitung 8 fördert. Am Verdampfer 7 kann
eine weitere Trocknung stattfinden.

0100074

Patentanspruch

Einrichtung zum Inertisieren von Erdgas unter Verwendung von
Rauchgas, mit einer Verbrennungsvorrichtung zum Erzeugen des
Rauchgases, mit einer Kühlvorrichtung zu dessen Kühlung und mit
einem Verdichter zum Verdichten des Rauchgases auf den für das
Zumischen zum Erdgas erforderlichen Druck, dadurch gekennzeichnet,
daß die Kühlvorrichtung (3) als Austreiber einer Absorptionskältevorrichtung (4) ausgebildet ist, deren Verdampfer (7) dem Verdichter (6) nachgeschaltet ist.

1/1

1 2 3 4 5 6 7 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 651 851 (GEBR. SULZER) | | C 07 C 9/04 |
| | | | C 07 C 7/20 // |
| | --- | | B 01 J 19/14 |
| A | DE-A-2 112 447 (BORSIG) | | C 10 L 3/00 |
| | ----- | | |

RECHERCHIERTE
SACHGEBIETE (Int. Cl. 3)

C 07 C 9/00
C 07 C 7/00
B 01 J 19/00
C 10 L 3/00

Der vorliegende Recherchenbericht wurde für alle Patentansprüche erstellt.

| Recherchenort | Abschlußdatum der Recherche | Prüfer |
|---|---|---|
| DEN HAAG | 26-10-1983 | VAN GEYT J.J.A. |